# EUROPEAN PATENT SPECIFICATION

(11) **EP 1 427 342 B1**
(45) Date of publication and mention of the grant of the patent: **03.09.2008**
(21) Application number: 02759475.3
(22) Date of filing: 26.08.2002
(51) Int. Cl.: A61B 17/70, A61F 2/44

(54) **BILATERAL LAMINOPLASTY IMPLANTS**
BILATERALE LAMINOPLASTIE-IMPLANTATE
IMPLANTS DE LAMINOPLASTIE BILATERALE

(30) Priority: 29.08.2001 US 942333
(43) Date of publication of application: 16.06.2004
(73) Proprietor: Synthes GmbH, 4436 Oberdorf (CH)
(72) Inventor: ANGELUCCI, Christopher, M., Schwenksville, PA 19473 (US); BOYER, Michael, L., II, Paoli, PA 19301 (US); PAUL, David, C., Phoenixville, PA 19460 (US); RYAN, Christopher, J., West Chester, PA 19380 (US); SINHA, Amit, Drexel Hill, PA 19026 (US); WALTHER, Martin, West Chester, PA 19380 (US)
(74) Representative: Jones Day
(86) International application number: PCT/US2002/027359
(87) International publication number: WO 2003/020143

(56) References cited:
- WO-A-01/70145
- DE-U- 9 413 778
- US-A- 5 980 572
- US-A- 5 989 289
- US-A- 6 143 033
- US-B1- 6 200 347
- US-B1- 6 277 149

## Description

### TECHNICAL FIELD

The present invention relates to a medical implant, and, more particularly, to an improved surgical implant for expanding the spinal canal to eliminate pressure on the spinal cord caused by an impinging vertebral bone.

### BACKGROUND OF THE INVENTION

Various medical conditions may result in a reduction of the area within the vertebrae available for the spinal cord. Spinal stenosis is one such condition involving the narrowing of the canal in the center of the spine through which the spinal cord and nerve roots run. Spinal stenosis may result when the ligaments of the spine thicken and calcify (harden from deposits of calcium salts), or when bones and joints enlarge, and osteophytes (bone spurs) form. A herniated (bulging) disk may also place pressure on the spinal cord or nerve root. Furthermore, diseased bone or tumors may result in an ingrowth into the spinal cord area. This decreases the space (neural foramen) available for nerve roots leaving the spinal cord.

Two surgical methods currently exist to create additional room in the spinal canal. The first is called a laminectomy, and involves removal of the lamina (roof) of one or more vertebrae. A limitation of the laminectomy procedure is that it involves removal of the supporting structures at the back of the vertebrae which align the spinal column. The result may be that a patient suffers some postural deformity. To prevent such postural problems, a graft may be installed between the ends of the removed bone to span the void and reinstate the necessary support. The second procedure is called a laminoplasty, in which the targeted vertebra is cut, spread apart and a graft is inserted to permanently enlarge the space. Unlike the laminectomy, typically no bone material is excised during the laminoplasty procedure. Two different laminoplasty procedures are currently used. The first is called the unilateral or "open door" laminoplasty in which one side (lamina) of the vertebra is cut all the way through, while the other side is cut only half way to create a hinge. The vertebral element is then rotated about the hinge, and the graft is inserted into the opening, increasing the opening of the spinal canal. The second procedure is called the bilateral or "French door" laminoplasty in which the midline of the vertebra (spinous process) is cut all the way through, and the lamina are cut half way through, creating two hinges. The vertebral element is then opened at the bisected spinous process, and a graft inserted into the opening, again increasing the opening of the spinal canal.

Various materials may be used for the grafts installed during laminoplasty procedures. U.S. Patent Nos. 6,080,157 to Cathro et al. and U.S. Patent No. 5,980,572 to Kim et al. disclose the use of titanium, ceramic and nylon inserts. Further, using allografts taken from long bones such as the femur, humerus, tibia and fibula, for spinal fusion procedures is known, as disclosed by U.S. Patent No. 5,728,159 to Stroever et al. Allografts, as such bone grafts are called, are removed from a donor and processed using known techniques to preserve the allograft until implantation. Allografts have mechanical properties which are similar to the mechanical properties of vertebrae even after processing. The benefit of such property matching is that it prevents stress shielding that occurs with metallic implants. Allografts, unlike magnetic metals, are also compatible with magnetic resonance imaging (MRI) procedures, allowing more accurate ascertainment of fusion. Furthermore, allografts are naturally osteogenic providing excellent long term fusion with the patient's own bone.

Several different spacer designs have been used in laminoplasty procedures to the present. For example, the Cathro patent discloses a metal, nylon or teflon spacer for use in a unilateral laminoplasty procedure. The Cathro spacer is a rectangular plate having shouldered edges which engage the ends of the cut lamina, and is held in place by a spring mechanism. The difficulty with the Cathro spacer is that its operation relies on the continued satisfactory operation of the installed spring. Further, the Cathro device provides little available area for the packing of fusion enhancing (i.e. osteogenic) material. The Kim patent discloses a spacer for use in a bilateral laminoplasty procedure. The Kim spacer consists of inner and outer trapezoidal segments joined together by a rectangular segment. The tapered surface of the inner trapezoidal segment is designed to conform to the inner surface of the split spinous process halves, while the taper of the outer segment is designed to assume the shape of the removed spinous process tip. The Kim spacer seats on the resulting flat surface of bone. Like the Cathro device, the Kim device provides little area in which to pack osteogenic material to facilitate bone-implant fusion. Neither the Cathro nor Kim device use allograft as a spacer material, which may result in reduced propensity for fusion and the possibility for stress shielding.

Accordingly, there is a need in the art to provide implants and methods for both laminectomy and unilateral and bilateral laminoplasty procedures, which provide excellent dimensional, strength and retention capability, which enhance fusion with the patient's own bone, which are easy to select, fit and install and which provide excellent compatability with post-operative imaging (MRI).

### SUMMARY OF THE INVENTION

The present invention is defined in claim 1 and provides implants for use in the spinal column. In one embodiment, the implants have a body portion having a length and configured to be insertable between first and second bone segments of the spinous process of a single vertebra. The body has an outer surface, and an inner surface defining a substantially hollow portion. The implants further have an inner side region having an inner side length, an outer side region having an outer side length, and first and second ends which communicate with the hollow portion. The first and second ends comprise bone engaging portions. At least one of the bone engaging portions comprises surface projections to reduce slippage between the bone engaging faces and the respective bone segment. The bone engaging portions are also be angled with respect to each other. The implant is configured so as not to protrude into the spinal canal when inserted between the first and second bone ends.

The implant inner side region comprises an angle with each of the bone engaging surfaces, and this angle may range from 50 to 70 degrees. Further, the inner side length of the implant may range from 6 to 10 millimeters. The perimeter of the outer surface of the implant may comprise a substantially geometric shape such as an ellipse or circle. An implant outer surface perimeter comprising an ellipse may have a width ranging from 10 to 11.5 millimeters (mm) and a depth ranging from 6.5 to 7.5 mm.

The implant may have bone engaging surfaces comprising surface projections to reduce slippage between the bone engaging portions and the respective bone segments. These surface projections may comprise saw tooth ridges, or they may be individual pyramidal teeth.

The implant bone engaging portions may comprise channels configured to accept the arms of a pair of distractor pliers. The implant body may have at least one hollow suture attachment portion to enable a surgeon to secure the implant to at least one of the first and second bone segments. The implant is formed of a bone allograft material and at least one of the bone engaging portions may be comprised of partially, substantially, or fully demineralized bone. The inner surface of the allograft implant may be defined by the intermedullary canal, or the inner surface may be configured so that the volume of the hollow portion is greater than the intermedullary canal.

A method for providing a desired space in the spinal canal comprises the steps of: cutting at least one segment of a vertebra all the way through to produce first and second cut bone ends; cutting at least one lamina partially to create a hinge; providing an implant having a body portion comprising a length and a longitudinal axis, the body portion having first and second ends and the first and second ends comprising bone engaging portions, where the implant is formed of a bone allograft material, and at least one of the bone engaging portions is comprised of partially, substantially, or fully demineralized bone; separating the first and second cut bone ends a sufficient distance to accept the implant; positioning the implant between the first and second cut bone ends; and contacting at least a portion of each of the first and second cut bone ends with the bone engaging portions. The step of cutting may comprise bisecting a spinous process. The method may further include the step of cutting a second lamina of the vertebra partially to create a second hinge.

A method for providing a desired distance between first and second cut bone ends of the spine comprises the steps of: cutting at least one segment of a vertebra to produce first and second cut bone ends; providing an implant having a body portion formed of bone allograft material with first and second ends; the first and second ends having bone engaging portions, the bone engaging portions each having an outer shell portion and a central region, the outer shell portion substantially surrounding the central region, wherein the outer shell portion is cortical bone and the center region is cancellous bone; separating the bone ends enough to accept the implant; placing the implant between the bone ends; and positioning the implant so as to contact at least a portion of each of the first and second cut bone ends with the bone engaging portions. The method may also include the step of providing an implant having bone engaging portions comprising surface projections to reduce slippage between the bone engaging portions and the bone ends. These surface projections may comprise saw tooth ridges or they may comprise individual pyramidal teeth. The step of cutting may comprise bisecting the spinous process. The cutting step may further comprise partially cutting both laminae adjacent to the spinous process.

### BRIEF DESCRIPTION OF THE DRAWINGS

The features and advantages of the implant and method of use will become more readily apparent from the following detailed description of the invention in which like elements are labeled similarly and in which:
FIGS. 1A, 1B and 1C are perspective, end and top views of the first embodiment of the implant, for use in a unilateral laminoplasty procedure;
FIGS. 2A and 2B are side and top views of the implant of FIG. 1 installed between the cut lamina segments of a vertebra during a unilateral laminoplasty procedure;
FIGS. 3A and 3B are a perspective view of a retaining plate of the present invention, and a side view of two such retaining plates installed over the implants of FIGS. 2A and 2B;
FIGS. 4A and 4B are perspective and side views of a second embodiment of the implant, a unilateral implant incorporating demineralized bone flaps;
FIGS. 5A, 5B and 5C are perspective, side and end views of a third embodiment of the implant, for use in a bilateral laminoplasty procedure;
FIGS. 6A and 6B are side and section views of the implant of FIG. 5 showing the incorporation of a channel to accept the corresponding arms of a set of distractor pliers used to install the implant;
FIG. 7 is a detail view of the end of the implant of FIG. 5B showing a preferred embodiment of the surface projections used to facilitate retention of the implant between cut spinous process segments.
FIGS. 8A, 8B and 8C are perspective, end and side views of a fourth embodiment of the implant, for use in a bilateral laminoplasty procedure;
FIG. 9A and 9B are front and top views of the implants of FIGS. 7 and 8 installed between the cut spinous process segments of a vertebra during a bilateral laminoplasty procedure;
FIGS. 10A, 10B and 10C are perspective, end and top views of a fifth embodiment of the implant, for use in a unilateral laminoplasty procedure;
FIGS. 11A, 11B and 11C are top, side and end views of an exemple of an implant, for use in a unilateral laminoplasty procedure; and
FIGS. 12A and 12B are perspective views of other examples of implants, for use in unilateral laminoplasty procedures.

### DETAILED DESCRIPTION OF THE PREFERRED EMBODIMENTS

Preferred embodiments, features and aspects of an implant adapted to be used in unilateral and bilateral laminoplasty procedures are described, in which a portion of a targeted vertebra is cut, the space available for the spinal cord and associated nerves is expanded, and an implant is installed between the cut segments of bone.

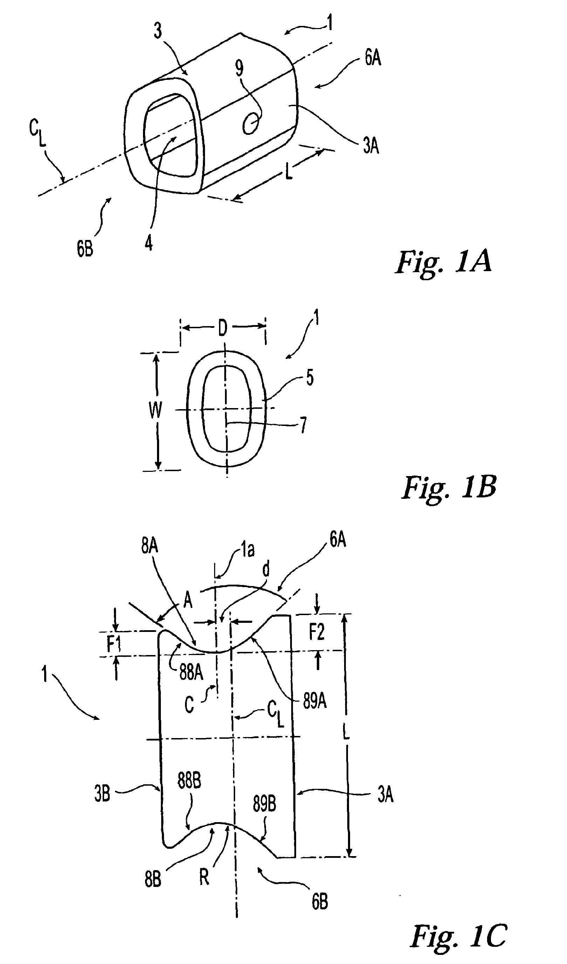

Referring more particularly to the drawings, FIGS. 1A, 1B and 1C show an implant for use in a unilateral or "open door" laminoplasty. The implant 1 has a longitudinal axis "CL," a length "L," a wall 5 defining an outside surface 3 and an inside surface 4, and first and second ends 6A, 6B. Inside surface 4 communicates with first and second ends 6A, 6B to define a hollow central region 7 of the implant. Outside surface 3 has an outer side region 3A and an inner side region 3B such that when the implant is installed between cut segments of lamina, outer side region 3A faces outward away from the spinal canal, while inner side region 3B faces inward toward the spinal canal. The implant 1 further has a depth "D" which is the distance between outer side region 3A and inner side region 3B. Implant 1 also has a width "W" which is the distance between opposing outer surfaces 3 measured along a drawn line perpendicular to a line defining the depth "D." Length "L" preferably should be between about 11.5 millimeters (mm) to about 15.5 mm; depth "D" preferably should be between about 5.5 mm to about 6.5 mm; and width "W" preferably should be between about 8.0 mm to about 9.5 mm.

The shape and size of outside surface 3 is not critical and, therefore, any implant configuration can be used preferably so long as the first and second ends 6A, 6B provide sufficient contact area with the lamina ends, and the implant 1 does not interfere with other anatomy, and does not intrude on the spinal cord space. In a preferred embodiment, however, the outside surface 3 is configured such that the shape of the implant, when viewed from the end, displays the form of a substantially geometric shape (e.g. ellipse, oval, circle, etc.). In this embodiment the exterior dimensions of the implant also approximate those of the outside surface of the cut lamina segments between which the implant is installed. Although implants having cross sections of greater or lesser proportion than the lamina to which they attach will function properly, for aesthetic purposes and in an attempt to minimize the amount of material introduced into a patient's body, the outer surface of the implant should preferably not extend beyond the outer surface of the adjoining bone.

In a further embodiment, the inside surface 4 of the implant 1 may be machined so that the hollow central region 7 approximates the configuration and geometry of the implant exterior (*i*.*e*. form an ellipse or oval shape). The hollow central region may be designed to be packed with osteogenic material such as bone chips, etc. to facilitate fusion of the implant with the patient's lamina. Preferably, the central region may be as large as possible to enhance fusion of the implant to the patient's lamina. The thickness of wall 45 preferably should be between about 1.00 to about 1.50 mm; more preferably about 1.25 mm. Preferably the thickness of wall 5 should not be less than about 1.0 mm to ensure the implant retains sufficient strength to withstand the stresses imparted on the spine.

The implant 1 is fabricated from allograft material preferably taken from a long bone (*e*.*g*. femur, tibia, fibula, humerus) and the inside surface 4 and hollow central region 7 may be defined by the intermedullary canal of the donor bone. The hollow center may be left as such, or the inner surface 4 may be machined, as with other implant materials, to maximize the space available for packing with osteogenic material. Again, the thickness of the implant wall 5, preferably is not reduced to less than about 1.00 mm.

During the unilateral laminoplasty procedure, the targeted lamina is cut in half and the segment attached to the spinous process is rotated or swung out to increase the area available for the spinal cord and associated nerves. Subsequent to this rotation, the lamina segments no longer reside along the same axis, but instead the ends are disposed at an angle with respect to each other. Implant 1 is substantially straight along its length, and so to accommodate this angular displacement of the lamina, first and second ends 6A, 6B incorporate arcuate cutouts 8A, 8B to grasp and retain the cut lamina segments. Viewed from the top of the implant (FIG. 1C), these arcuate cutouts 8A, 8B are generally concave and may be circular in shape, or they may consist of a cutout spanning an obtuse angle and converging to a small radius at the crotch of the first and second ends 6A, 6B. Arcuate cutouts 8A, 8B have a centerline 1a which runs parallel to the longitudinal axis of the implant 1. The centerline 1a of the arcuate cutouts may be coexistent with the longitudinal axis of the implant 1, or it may be offset with respect to that axis to further improve retention of the cut and displaced lamina ends. In a further embodiment, the centerlines 1a of the arcuate cutouts may each be offset on an opposite side of the implant centerline to facilitate retention of the implant in cases where the angle between the cut and spread lamina is more severe, such as when the surgeon spreads the lamina segments as wide as possible to provide maximum additional space for the spinal cord and associated nerves.

In the preferred embodiment, shown in FIG. 1C, each arcuate cutout 8A, 8B comprises first angled faces 88A, 89A and second angled faces 88B, 89B, respectively, which meet at crotch "C" to form a face angle "A." Preferably, face angle A is about 100 degrees. Crotch radius "R," comprises the transition between the first and second angled faces. Crotch radius "R" is preferably about 2 mm. Each arcuate cutout further comprises first and second face depths "F1" and "F2." The first and second face depths are a measure of the depth of the crotch relative to the inner side region 3B and outer side region 3A of the implant, and will be different lengths whenever the centerline 1a of the arcuate cutout is offset from the centerline "CL" of the implant 1. Preferably first face depth "F1" is about 1.25 mm, and second face depth "F2" is about 1.5 mm. Each arcuate cutout 8A, 8B also has a centerline offset "d," which is the degree to which the arcuate cutout 8A, 8B is shifted from the centerline "CL" of the implant 1. Preferably, the centerline offset "d" is from about 0 to 2.5 mm toward the inner side region 3B of implant 1. The face depth "F1" of the first and 6A of the implant 1 may be the same or different than the face depth "F1" of the second end 6B. Likewise, the face depth "F2" of the first end 6A may be the same or different than the face depth "F1" of the second end 6A.

In a further embodiment of the implant comprising allograft material, first and second ends 8A, 8B may comprise regions of demineralized cortical bone to further facilitate fusion of the implant to the lamina. Preferably the demineralized bone portion comprises the entire surface of each first and second end 6A, 6B of the implant 1. Preferably, the depth of the demineralized portion will be up to about 2 mm.

The implants further may incorporate at least one suture hole 9 in the implant wall 5 to allow the surgeon the option of suturing the implant to the cut lamina ends. These suture holes 9 may vary in number and size, with the only limitation being that they should not be so large or numerous as to compromise the strength or integrity of the implant.

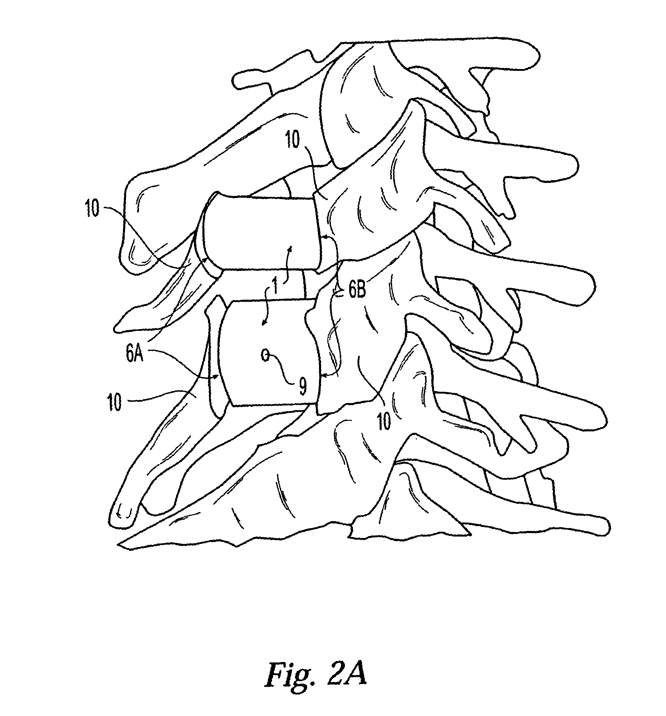

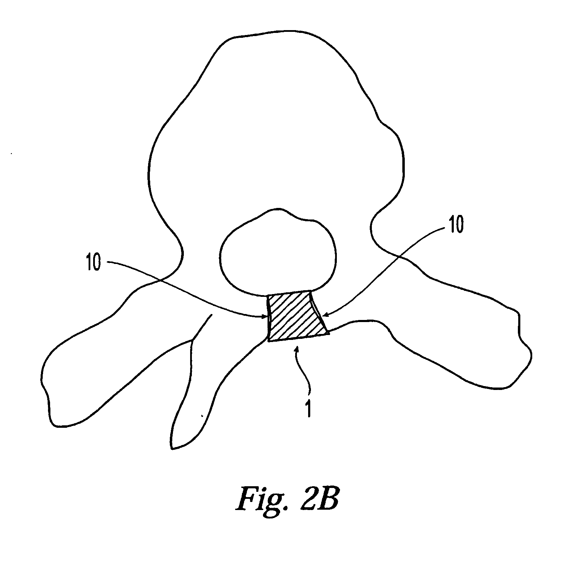

FIGS. 2A and 2B are side and top views of the implant of FIG. 1 installed in a patient between the cut lamina ends in a unilateral laminoplasty procedure. In FIG. 2A two different sized implants 1 are installed on the cut lamina segments 10 of adjacent vertebrae, to illustrate application of the implant design to bones of different size. FIG. 2B shows the interaction between the implant and the cut vertebra segments 10.

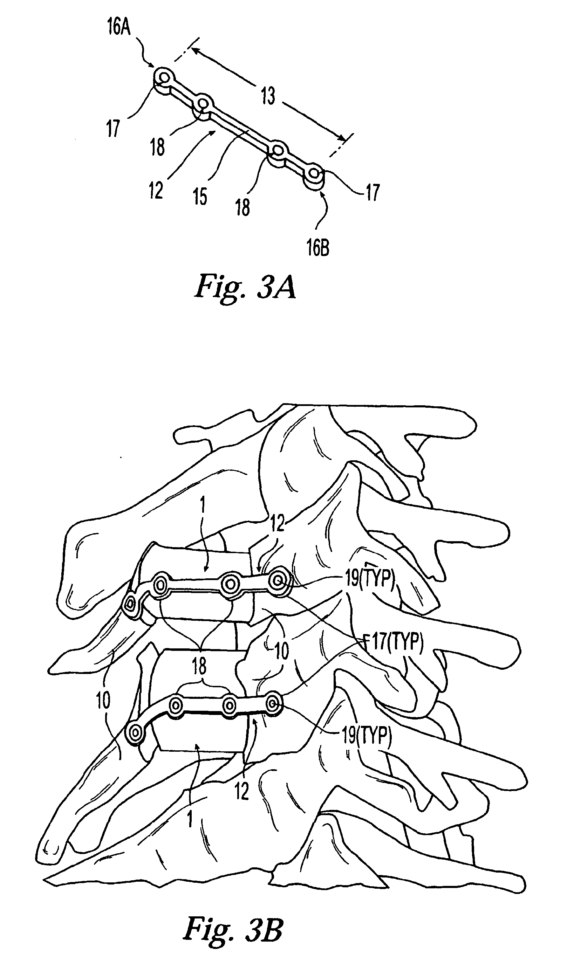

The design of the bone engaging ends 6A, 6B of the implants 1 are sufficient to ensure retention of the implants 1 between the cut ends of lamina 10. Some surgeons, however, desire an additional measure of assurance that the implants 1 will not loosen or otherwise be expelled from between the lamina ends 10. The implant, therefore, provides for the optional installation of a plate 12 to be secured over an installed implant in a unilateral laminoplasty procedure. FIG. 3A is a perspective view of a plate 12 which may be installed to secure the implant 1 of FIGS. 1 & 2, to ensure the implant 1 is not expelled from the cut lamina ends 10. Plate 12 has a length 13, a thickness 14 and a body portion 15 with first and second ends 16A, 16B comprising bone engaging portions 17 and implant engaging portions 18. As shown in FIG. 3A the bone engaging portions 17 and implant engaging portions 18 may consist of the holes adapted for receiving bone screws 19 or hooks 20 (not shown) capable of grasping bone screws installed in the lamina and/or implant. Each side of plate 12 may have one or more bone engaging portions 19 and one or more implant engaging portions 18. In a further embodiment the plate 12 may be flexible to allow the surgeon to form it to the individual contour of the patient's spine, thereby achieving a tight fit between components. The plates may be fabricated from a biocompatable metal or other material known in the art that would be suitable for long term retention of an implant 1.

Instead of a single plate 12, smaller plates without connecting body portion 15 may be utilized, each plate comprising at least one bone engaging portion 17 and one implant engaging portion 18.

FIG. 3B is a side view of the implants 1 installed in FIG. 2A, further showing the installation of optional plates 12 of FIG. 3A. Bone screws 19 are installed to secure the plates 12 to both the respective opposing lamina segment 10, and the implant. In this embodiment, bone screws are also installed in the screw holes 18 of the implant engaging portion, to secure the plates to the implants 1. Also in this embodiment, the plates are flexible and are bent to assume the varying contour of the lamina segments and the implant. More than one optional plate may be used to secure the implant to the lamina.

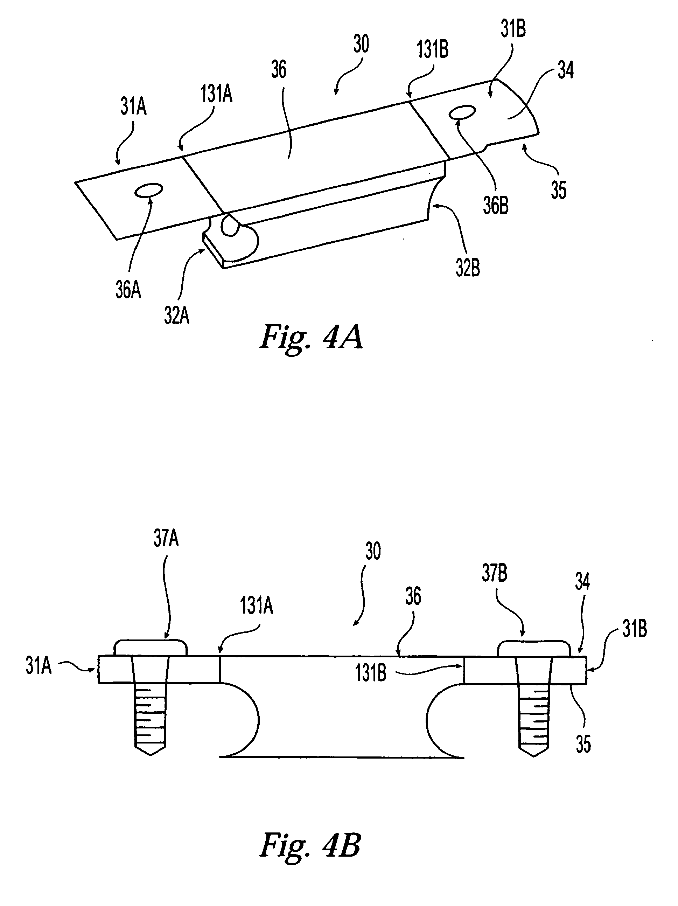

FIGS. 4A and 4B show perspective and side views of an allograft implant 30 which incorporates the design features of the implants of FIG. 1, but which further includes a pair of bone flaps 31A, 31B disposed at first and second ends 32A, 32B of the implant 30. These bone flaps are used to secure the implant 30 to the respective cut ends of lamina in a unilateral laminoplasty procedure. At least a portion of each flap comprises demineralized bone. Demineralization of the flaps, but not the implant, provides the implant with flexible attachment points which may be contoured to conform to the shape of the adjacent lamina. Bone flaps 31A, 31B comprise thin, flat, rectangular segments of allograft having an outer surface 34 and a bone engaging surface 35. The outer surfaces 34 of the flaps preferably are the same width as, are contiguous with, and extend axially like wings from the outer surface 36 of the implant 30. In a preferred embodiment, bone flaps 31A, 31B are machined from the same segment of donor bone as implant 30. At least a portion of flaps 31A, 31B may be demineralized using any commercially acceptable process (e.g. hydrochloric acid bath, etc.) that will render the resulting flaps flexible. Flaps 31A, B are provided with holes 36A, 36B suitable for receiving bone screws 37A, 37B which are used to secure the bone flaps 31A, 31B and implant 30 to the adjacent cut lamina ends.

In another embodiment, these bone flaps may not be demineralized, but instead each bone flap may comprise a notch 131A, 131B in the respective region where the bone flaps 31A, 31B connect to the implant 30. Notches 131A, 131B may be any type of notch or reduction in the thickness of the bone flap appropriate to provide flexibility for placing the flaps on the adjacent laminae surfaces, while retaining the requisite strength to ensure the bone flaps will not separate from the implant during installation.

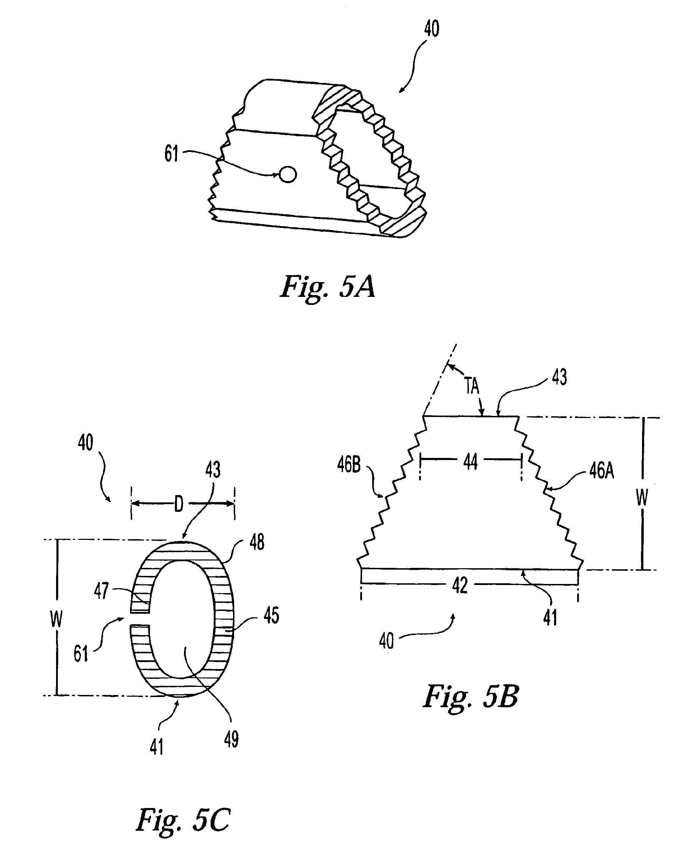

FIGS. 5A, 5B and 5C show an embodiment of an implant for use in a bilateral or "french door" laminoplasty procedure, in which the spinous process of a targeted vertebra is bisected along the sagittal plane and the segments separated to enlarge the spinal canal. The implant 40 has a wall 45 having an inside surface 47 and an outside surface 48, and first and second ends 46A, 46B. The outside surface 48 has an outer side region 41 having an outer side length 42 and an inner side region 43 having an inner side length 44. Inside surface 47 communicates with first and second ends 46A & 46B to define a hollow central region 49 of the implant. The implant 40 has a generally trapezoidal shape when viewed from the side (FIG. 5B), and inner side region 43 forms angle "TA" with respect to the first and second ends 46A, 46B. This trapezoidal configuration allows the implant first and second ends 46A, 46B to conform to the cut, angled surfaces of the spinous process segments to which the implant will eventually fuse. Inner side length 44 preferably is from between about 6.0 mm to about 10 mm, and angle "TA" preferably is from between about 50 to about 70 degrees. The implant 40 further has a width "W" which is the distance between the outer side region 41 and the inner side region 43. Implant 40 also has a depth "D" which is the distance between opposing outside surfaces 48 measured along a line drawn perpendicular to a line defining the width "W." Width "W" preferably should be between about 10 mm to about 11.5 mm; and depth "D" preferably should be between about 6.5 mm to about 7.5 mm.

The shape and size of outside surface 48 is not critical and, therefore, any implant external configuration can be used preferably so long as first and second ends 46A, 46B provide sufficient contact area with the cut spinous process segments, does not project out from between the bone segments so far as to interfere with other anatomy, and does not intrude on the spinal cord space For aesthetic purposes and in an attempt to minimize the amount of new material introduced into a patient, however, the outside surface 41 of the implant 40 should preferably not extend beyond the outside surface of the cut spinous process segments. In a preferred embodiment the outside surface 41 of the implant 40 is configured such that the outside surface 41, when viewed from the end, displays the form of a substantially geometric shape (e.g. ellipse, oval, circle, etc.) (FIG. 5C).

In a further embodiment, the inside surface 43 of the implant 40 may be machined so that the hollow central region 49 approximates the configuration and geometry of the implant outside surface 41 (i.e. an ellipse or oval). The hollow central area is designed to be packed with osteogenic material such as bone chips, etc. to facilitate fusion of the implant with the patient's cut spinous process segments. Preferably, this center area may be made as large as possible to facilitate the fusion process.

The thickness of wall 45 preferably should be from between about 1.00 to about 1.50 mm; more preferably about 1.25 mm. Preferably the thickness of wall 45 should not be less than about 1.0 mm to ensure the implant retains sufficient strength to withstand the stresses imparted on the spine associated with daily living.

The implant 40 is fabricated from allograft material preferably taken from a long bone (e.g. femur, tibia, fibula, humerus). The implant should incorporate a hollow region, and the inside surface 44, should be formed to maximize the space available for packing with osteogenic material while maintaining adequate wall thickness. The inside surface 44 and hollow center 49 may be defined by the intermedullary canal of the donor bone. The allograft may be left in this state, and the hollow central region 49 packed with osteogenic material. Preferably, however, the inside surface 44 of the allograft will be machined and the hollow central region 49 enlarged to maximize the space available for packing with osteogenic material.

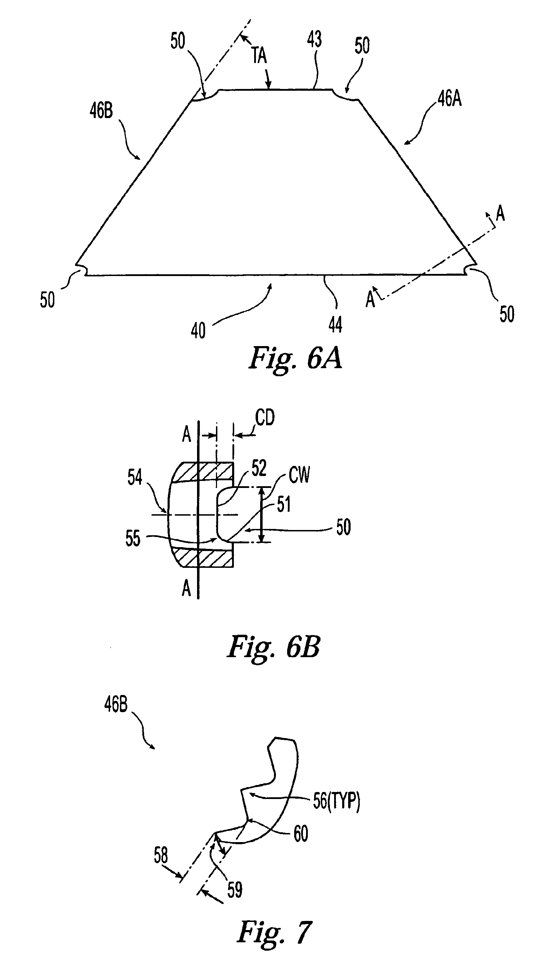

FIGS. 6A and 6B show first and second ends 46A, 46B of implant 40 each incorporating a channel 50 to accept the corresponding arms of a set of distractor pliers (not shown) which may be used to separate the bisected spinous process segments during the bilateral laminoplasty procedure. Each channel 50 has two sidewalls 51 each having a depth "CD", a bottom surface 52 having a width "CW" and a centerline 54 which is formed by a line extending along the implant 40 from inner side region 43 to outer side region 41. Preferably, each channel 50 may incorporate a radiused transition 55 between the sidewalls 51 and the bottom surface 52. In a further preferred embodiment, the channel runs from the inner side region 43 to the outer side region 41 of each end 46A, 46B of the implant. The specific dimensions of the channels is not critical, but should be configured to accept the distractor arms used during the distraction and insertion portion of the procedure. Preferably, the channel bottom surface width "CW" is about 4 mm, and the sidewall depth "CD" is about 1 mm.

FIG. 7 shows a further embodiment of bilateral laminoplasty implant 40, in which first and second ends 46A, 46B comprise surface projections to improve pre-fusion retention of the implant 40 between respective cut spinous process segments. In a preferred embodiment, a plurality of saw-tooth serrations 56 having a height 58 and a tooth angle 59 are provided. Preferably the serrations are oriented to run vertically when the implant 40 is installed in the patient. Height 58 and tooth angle 59 are defined with respect to the respective planes formed by implant first and second ends 46A, 46B. Height 58 is measured from the trough 60 of each serration, while tooth angle is measured from the plane formed by the implant first and second ends 46A, 46B. Preferably, height 58 is about 0.5 mm, tooth angle 59 is about 45 degrees, and the distance between troughs 60 is about 1.2 mm. While these dimensions and profile are preferred, other suitable surface profiles (e.g. pyramidal teeth, etc.) may be used to ensure implant retention.

In a further embodiment of the implant 40 comprising allograft material, first and second ends 46A, 46B may comprise regions of partially, substantially, or fully demineralized cortical bone to further facilitate fusion of the implant to the lamina. Preferably the demineralized bone portion may comprise the entire surface of each first and second ends 46A, 46B of the implant 40. Preferably the depth of the demineralized portion of will be up to about 2 mm.

The implant 40 may also incorporate a plurality of sutures holes 61 (see FIG. 5C) formed through the implant wall 45 to allow the surgeon to secure the implant to the cut spinous process segments. These suture holes 61 may vary in number, size and position, with the only limitation being that their size, position and number preferably should not compromise the strength and integrity of the implant.

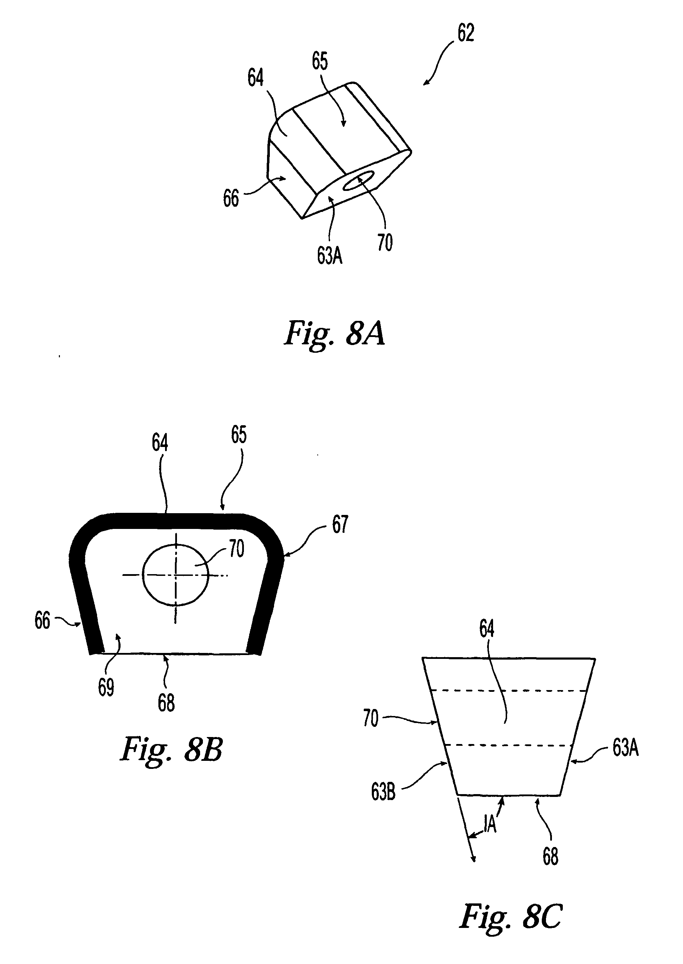

FIGS. 8A, 8B and 8C show a further embodiment of an implant for use in a bilateral laminoplasty procedure. Implant 62 has a first and second ends 63A, 63B, an inner side region 68, an outer side region 65, and sides 66 and 67. The implant 62, like the implant of FIG. 5, has a generally trapezoidal shape when viewed from the side (FIG. 8C). Again, this trapezoidal configuration allows the implant first and second ends 63A, 63B to conform to the cut, angled surfaces of the spinous process segments to which the implant will eventually fuse. As such, inner side 68 forms angle "IA" with respect to the first and second ends 63A, 63B. In this embodiment, the implant 62 is an allograft, comprising "tri-cortical" bone taken from the crest of the ilium region of the pelvis. Harvesting bone from this segment of the pelvis provides an implant which naturally comprises a thin region 64 of cortical bone on outer side 65, and sides 66 & 67. The inner side 68 of the implant, as well as the implant body portion 69 comprise cancellous bone. This combination of bone types allows the surgeon to exploit both the good strength characteristics of cortical bone, and the good osteogenic characteristics of cancellous bone in a single implant. In a further embodiment, the implant 62 comprises a cavity 70 which communicates with implant first and second ends 63A & 63B, and which may be used for packing osteogenic material to promote fusion between the implant and the cut spinous process segments.

In a preferred embodiment of the implant 62 of FIG. 8, the implant first and second ends 63A, 63B comprise surface projections to improve pre-fusion retention of the implant 62 between respective cut spinous process segments. Saw-tooth serrations, similar to those illustrated and described with regard to the implant of FIG. 5, may be provided. Again, other suitable surface profiles (e.g. pyramidal teeth, etc.) may also be provided to ensure implant retention.

In a further embodiment of the implant 62 comprising allograft material, first and second ends 63A, 63B may comprise regions of partially, substantially, or fully demineralized cortical bone to further facilitate fusion of the implant to the lamina. Preferably the demineralized bone portion may comprise the entire surface of each first and second ends 63A, 63B of the implant 62. Preferably, the depth of the demineralized portion of will be up to about 2 mm.

In another embodiment, the implant 62 may incorporate a plurality of sutures holes (not shown) similar to those shown in FIG. 5C, to allow the surgeon to secure the implant to the cut spinous process segments. These suture holes may vary in number, size and position, with the only limitation being that their number, size and position should not compromise the strength and integrity of the implant.

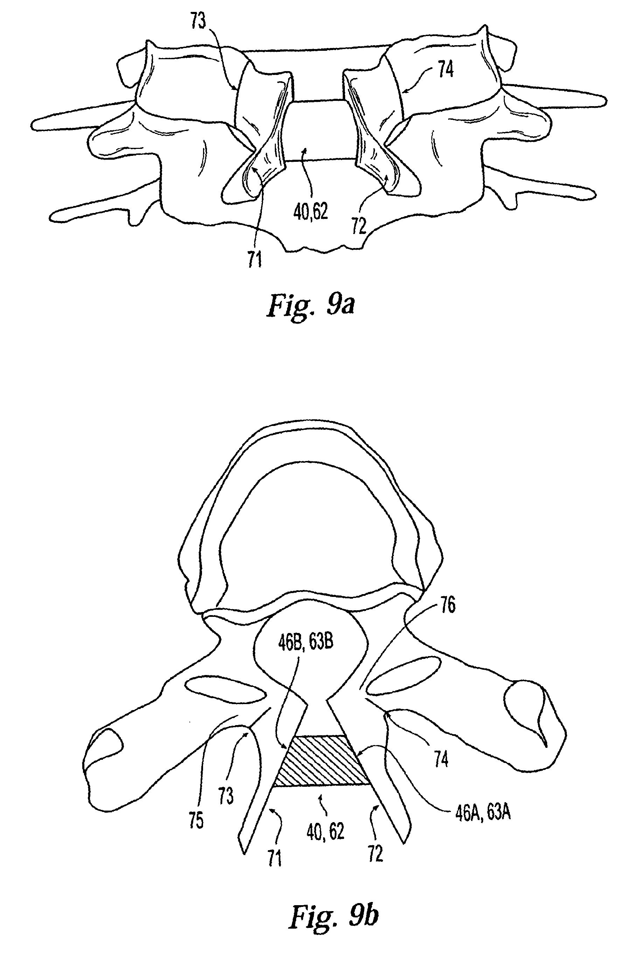

FIGS. 9A and 9B are front and top views of either trapezoidal implants 40, 62 of FIGS. 5, 8 installed in a patient. First and second ends 46A, 46B, 63A, 63B of implant 40, 62 contact cut spinous process segments 72 and 71 respectively. Hinge cuts 73 and 74 in lamina 75, 76 enable the spinous process segments to be "swung out" by the surgeon to facilitate insertion of the implant 40, 62 therebetween.

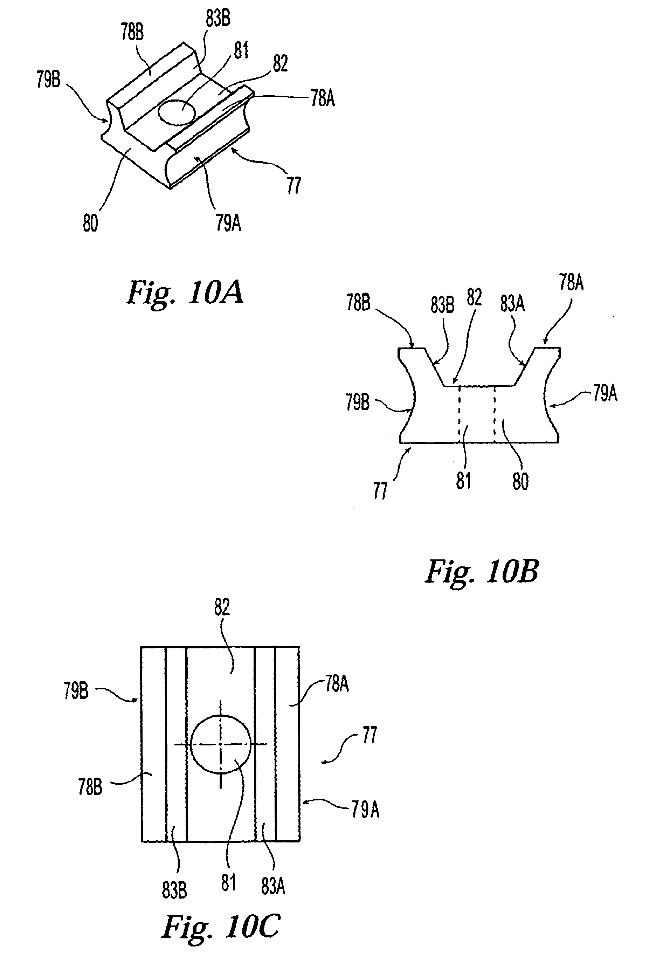

FIGS. 10A, 10B and 10C show a further embodiment of an implant adapted for use in a unilateral laminoplasty procedure. Implant 77 comprises first and second plate portions 78A, 78B for connecting to the opposing segments of cut lamina produced during a unilateral laminoplasty procedure. First and second plate portions 78A, 78B are connected by an intermediate portion 80. The plate portions further comprise respective first and second bone engaging portions 79A, 79B which are configured to engage the opposing cut lamina segments. In a preferred embodiment, first and second bone engaging portions 79A, 79B comprise arcuate surfaces for engaging and cradling the respective cut lamina ends. Arcuate surfaces are particularly suited for this purpose because their concave shape can engage and retain lamina segments residing along different axes, a phenomenon which occurs during the unilateral laminoplasty procedure when a single lamina is cut and the resulting segments are swung out to enlarge the area available for the spinal cord. The swinging out process results in an angle being formed between the segments, and it is this misalignment which the arcuate surfaces of the bone engaging portions 79A & 79B accommodate.

In a further embodiment, the thickness of the intermediate portion 80 may be smaller than the height of the first and second plate portions 78A, 78B.

Implant 77 is fabricated from allograft material and preferably from cortical bone.

In a further embodiment of the implant 77 comprising allograft material, first and second bone engaging portions 79A, 79B may comprise regions of partially, substantially, or fully demineralized bone to further facilitate fusion of the implant to the lamina segments. Preferably the demineralized bone portion may comprise the entire surface of each first and second bone engaging portions 79A, 79B. Preferably, the depth of the demineralized portion will be up to about 2 mm.

In another embodiment, the implant 77 may incorporate suture hole 80 to allow the surgeon to secure the implant to the cut spinous process segments. Additional suture holes (not shown) may be provided, and may vary in number, size and position, with the only limitation being that their size, position and number preferably should not compromise the strength and integrity of the implant 77.

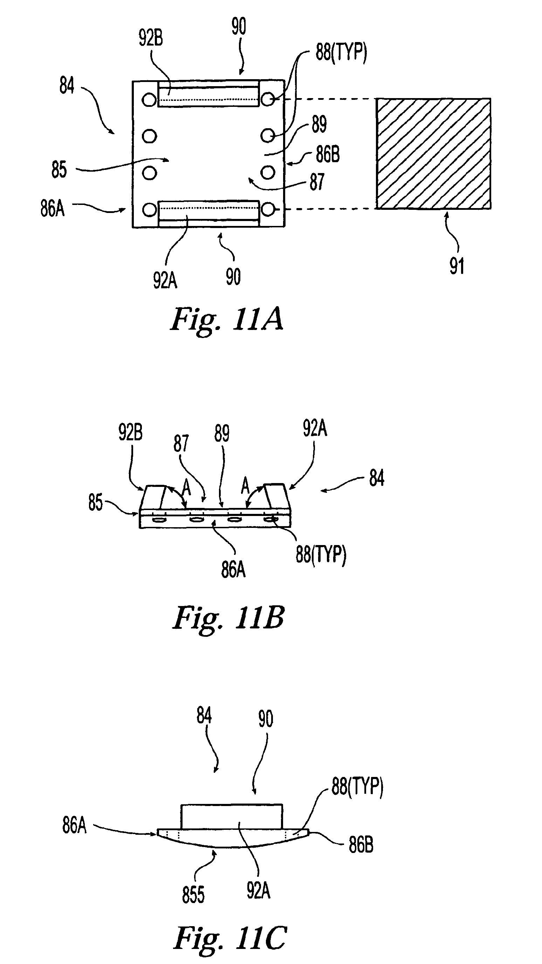

FIGS. 11A, 11B and 11C show an example of an implant adapted for use in a unilateral laminoplasty procedure. Implant 84 comprises a plate portion 85 having bone engaging portions 86A, 86B, a graft engaging portion 87, and an allograft 91. Bone engaging portions 86A, 86B further comprise a plurality of suture holes 88 configured to allow the surgeon to secure the cut lamina segments to bone engaging portions 86A, 86B Graft engaging portion 87 comprises a graft seating surface 89 and a graft retaining portion 90 configured to retain a correspondingly shaped allograft 91 for engaging the opposing cut lamina segment. In a preferred embodiment, graft retaining portion 90 comprises two raised tabs 92A, 92B, each residing along at least a portion of opposing ends of graft seating surface 89. In a preferred embodiment, raised tabs 92A, 92B are angled slightly toward the center of graft seating surface 89 so as to facilitate retention of allograft 91. Preferably the angle "A" between raised tabs 92A, 92B and graft seating surface 89 will be from about 70 to about 80 degrees; more preferably this angle will be about 75 degrees. Plate portion 85 further comprises a bottom surface 855. When installed, graft 91 comprises the inner side surface of the implant (i.e. the surface which is closest to the spinal canal), while plate bottom surface 855 comprises the outer side surface of the implant (i.e. the surface which faces away from the spinal canal). In a preferred embodiment, bottom surface 855 comprises a convex shape which assumes the rounded contour of the lamina segments. Preferably, this convex surface has a radius of about 18 mm.

Plate portion 85 may be fabricated from any biocompatable metal (e.g. titanium, stainless steel, etc.) or polymer, or it may be made of allograft material. If allograft is used, the plate portion 85 may be fabricated from cortical bone. Graft 91 preferably may be comprised of a cancellous type bone material to facilitate fusion of the implant to the patient's lamina.

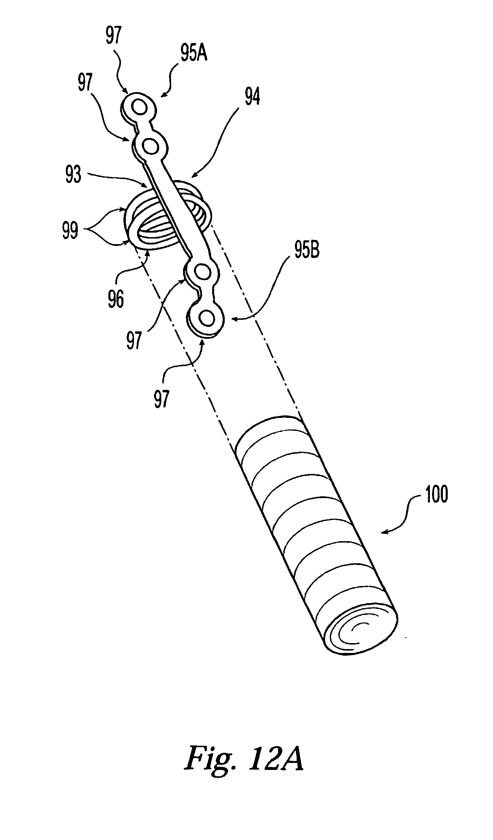

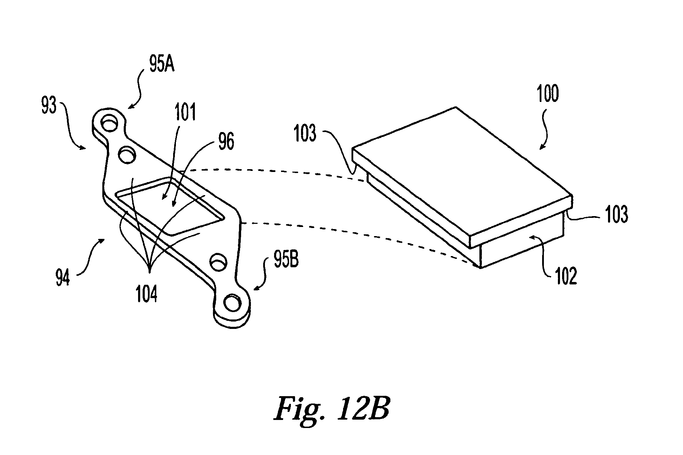

FIGS. 12A and 12B show implant examples comprising plates configured to attach directly to the opposing cut segments of lamina produced during a unilateral laminoplasty. These plates are further configured to capture segments of allograft and to engage these segments with the opposing cut segments of lamina to facilitate fusion between the implant and the patient's bone. Plate 93 comprises a body portion 94 having a longitudinal axis and first and second ends 95A, 95B, and a graft retaining portion 96, midway between the ends 95A, 95B, preferably approximately midway between ends 95A, 95B. First and second ends 95A, 95B each comprise a bone engaging portion 97. In a preferred embodiment the bone engaging portion at each first and second end comprises at least one hole suitable for receiving a bone screw 98 (not shown). The bone screws are then used to secure the plate 93 to each opposing segment of lamina. In a further embodiment the bone engaging portions may be hooks capable of grasping bone screws that are installed in the lamina segments.

In the example shown in FIG. 12A, the graft retaining portion 96 comprises a plurality of deformable fingers 99 which are initially arrayed flat along an axis perpendicular to the longitudinal axis of the plate 93. These fingers 99 are capable of being deformed to cradle an allograft 100, preferably cylindrical in shape. Allograft 100 preferably has a length sufficient to engage the cut ends of lamina upon installation, and a diameter of size sufficient to be captured by the deformed fingers 99 of the plate 93.

In the example of FIG. 12B, plate 93 has a graft retaining portion 96 which comprises a hollow region 101, preferably rectangular in shape. A correspondingly configured allograft of cancellous bone is provided having a body 102 capable of being received within the hollow region 101, and further having shoulders 103 which extends beyond the hollow region to contact seating surface 104. Shoulders 103 of allograft 100 may be secured to plate 93 using a bone screw 98 placed through bone engaging portion 97.

The plate 93 may be flexible to allow the surgeon to form the body 94 to the individual contour of the patient's spine, thereby achieving a tight fit between components. The plate 93 may be fabricated from a biocompatable metal or other material known in the art that would be suitable for long term retention of an implant and graft.

A method of using an allograft implant according to any of the embodiments shown in FIGS. 1A, 5A, 8A, 10A or 11A which further has partially, substantially, or fully demineralized end segments to promote fusion between opposing segments of lamina or spinous process produced during a unilateral or bilateral laminoplasty procedure comprises the steps of cutting a targeted lamina or spinous process as required for either a unilateral or bilateral laminoplasty procedure, separating the resulting segments of bone a sufficient distance to allow for insertion of an appropriately sized allograft implant, providing an allograft implant having bone engaging surfaces which comprise partially, substantially, or fully demineralized cortical bone to a depth of up to about 2 mm, and contacting the allograft implant bone engaging surfaces with respective cut segments of lamina or spinous process. This method may be augmented, in the case of a unilateral laminoplasty, to include the additional step of installing a plate over the allograft implant to further assist retention of the implant between the bone segments. Where such a plate is provided, the additional steps of providing bone screws or other fasteners to attach the plate to the opposing segments of bone and/or to attach the plate to the implant, may further be included.

A further refinement of the above method comprises providing an allograft implant according to the above method, which implant further has partially, substantially, or fully demineralized bone flaps capable of receiving bone screws. Providing such an implant allows the surgeon to affirmatively secure the implant to the cut lamina segments, preferably without the need for a separate plate.

A method of installing a tri-cortical allograft implant as part of a bilateral laminoplasty procedure comprises the steps of bisecting a targeted spinous process, providing hinge cuts on both adjacent lamina sufficient to allow the spinous process segments to be spread apart, separating the spinous process segments to allow for insertion of an appropriately sized allograft implant, providing an allograft implant having first and second angled bone engaging surfaces which approximate the angle between the bisected and spread spinous process segment cut surfaces, the allograft implant comprising cancellous bone material having a thin outer layer of cortical bone surrounding the cancellous bone, and which cortical bone layer is in communication with the first and second engaging surfaces so as to support the compressive stresses imparted by the cut spinous process segments.

A method of using only a screwed plate to maintain the distance between bone ends produced during a unilateral or bilateral laminoplasty procedure comprises the steps of cutting a targeted lamina or spinous process as required for the respective laminoplasty procedure, separating the cut bone segments to increase the space available for the spinal canal and associated nerves, providing an appropriately sized plate having first and second ends, wherein each end is configured to allow engagement with the surface of the lamina opposite the surface of the spinal canal and adjacent the cut bone end, and securing first and second ends of the plate to the adjacent bone segments.

In a refinement of the method, each first and second end of the plate will have at least one recess suitable for receiving a bone screw, wherein the plate is secured to the adjacent cut bone ends using bone screws. In a further refinement, two plates may be provided to attach to the adjacent cut bone ends.

Accordingly, it should be understood that the embodiments disclosed herein are merely illustrative of the principles of the invention. Various other modifications may be made by those skilled in the art which will embody the principles of the invention and fall within the scope of the appended claims.

## Claims

1. An implant (1) for use in maintaining a desired distance between first and second bisected bone ends of a spinal column, said implant comprising:
a body portion having a length (L) and configured to be insertable between first and second bone ends of the spinous process of a single vertebra,
the body portion having an outer surface (3), and an inner surface (4) defining a substantially hollow portion (7), the body portion further having an inner side region (3B) having an inner side length, and first (6A) and second (6B) ends which communicate with said hollow portion, the first (6A) and second (6B) ends comprising bone engaging portions, at least one of the bone engaging portions comprises surface projections to reduce slippage between the bone engaging portions and the respective said bone segment,
wherein said bone engaging portions are angled with respect to each other and said implant is configured so as not to protrude into the spinal canal when inserted between the first and second bone ends, **characterised in that** said body portion is substantially tubular and is formed of bone allograft material.

2. The implant of Claim 1, wherein the intersection between the inner side region and each of the bone engaging portions comprises an angle.

3. The implant of Claim 2, wherein the angle ranges from about 50 to about 70 degrees, and the inner side length ranges from about 6 to about 10 millimeters.

4. The implant of any one of the preceding Claims 1-3, wherein the perimeter of the outer surface (3) of the implant is a substantially geometric shape.

5. The implant of Claim 4, wherein the geometric shape is an ellipse having a width (W) and a depth (D) or the geometric shape is a circle.

6. The implant of Claim 5, wherein the width ranges from about 10.0 to about 11.5 millimeters and the depth ranges from about 6.5 to about 7.5 millimeters.

7. The implant of any one of the preceding Claims 1-6, wherein the surface projections comprise saw tooth ridges, or wherein the surface projections comprise individual pyramidal teeth.

8. The implant of any one of the preceding Claims 1-7, wherein each of the first and second ends further comprises a channel configured to accept the arms of a pair of distractor pliers.

9. The implant of any one of the preceding Claims 1-8, wherein the body portion further comprises at least one hollow suture attachment portion (9) to enable a surgeon to secure the implant to at least one of said first and second bone segments.

10. The implant of any one of the preceding Claims 1-9 wherein at least one of the first and second bone engaging portions is comprised of demineralized bone.

11. The implant of Claim 10, wherein said inner surface (4) is defined by the intermedullary canal of the donor bone.

12. The implant of Claim 10 or 11, wherein said inner surface is configured such that the volume of said substantially hollow portion is greater than the intermedullary canal of the donor bone.

13. The implant of any one of Claims 1-9, wherein at least a portion of the implant is fabricated of biocompatable metal, or of biocompatable polymer.

14. The implant of Claim 1, wherein the substantially tubular body portion further comprises a width (W) and a depth (D) and wherein at least one of the first and second bone engaging portions is comprised of a demineralized allograft material.

15. The implant of Claim 14, wherein the body portion further comprises a wall having an outer surface, and an inner surface defining a substantially hollow portion, wherein the hollow portion is in communication with the first and second ends.

16. The implant of Claim 15, wherein said hollow portion (7) is defined by the intermedullary canal of the donor bone.

17. The implant of Claims 15 or 16, wherein said inner surface (4) is configured such that the volume of said substantially hollow portion (7) is greater than the intermedullary canal of the donor bone.

18. The implant of any one of the preceding Claims 14-17, wherein the at least one of said first and second bone engaging portions further comprises surface projections configured to retain said implant within the first and second bone segments.

19. The implant of Claim 14 wherein the surface projections comprise saw-tooth ridges, or wherein the surface projections comprise individual pyramidal teeth.

20. The implant of any one of Claims 14-19, wherein intersection between the inner side region (3B) and each of the first and second bone engaging portions comprises an angle.

21. The implant of Claim 20, wherein the angle ranges from about 50 to about 70 degrees, the inner side length ranges from about 6 to about 10 millimeters, the width (W) ranges from about 10 millimeters to about 11.5 millimeters, and the depth (D) ranges from about 6.5 millimeters to about 7.7 millimeters.

22. The implant of Claim 1, wherein the at least one bone engaging portion further comprises an outer shell portion substantially surrounding a center region, wherein the outer shell portion is cortical bone and the center region is cancellous bone.

23. The implant of Claim 22, wherein the surface projections comprise saw tooth ridges, or wherein the surface projections comprise individual pyramidal teeth.

24. The implant of Claim 22 or 23, wherein at least one of the first and second bone engaging faces is comprised of demineralized bone.

25. The implant of Claim 22 or 23, wherein the intersection between the inner side and the at least one bone engaging portion comprises an angle.

26. The implant of Claim 25, wherein the angle ranges from about 50 to about 70 degrees, and the inner side length ranges from about 6 to about 10 millimeters.

27. The implant of any one of the preceding Claims 22-26, wherein the body portion further comprises a hollow suture attachment portion (9) to enable a surgeon to secure the implant to at least one of said first and second bone segments.

## Patentansprüche

1. Implantat (1) zur Verwendung beim Aufrechterhalten eines gewünschten Abstands zwischen einem ersten und einem zweiten getrennten Knochenende einer Wirbelsäule, wobei das Implantat umfasst:
ein Körperteil, das eine Länge (L) hat und dazu ausgelegt ist, zwischen einem ersten und einem zweiten Knochenende des Dornfortsatzes eines einzelnen Wirbels einsetzbar zu sein,
wobei das Körperteil eine Außenoberfläche (3) und eine Innenoberfläche (4) aufweist, die einen im Wesentlichen hohlen Teil (7) definiert, wobei das Körperteil ferner einen inneren Seitenbereich (3B), der eine innere Seitenlänge aufweist, und ein erstes (6A) und ein zweites (6B) Ende hat, die mit dem hohlen Teil in Verbidung stehen, wobei das erste (6A) und das zweite (6B) Ende Knocheneingriffsteile aufweisen, wobei mindestens einer der Knocheneingriffsteile Oberflächenfortsätze aufweist, um ein Verschieben zwischen den Knocheneingriffsteilen und dem entsprechenden Knochenabschnitt zu verringern,
wobei die Knocheneingriffsteile in einem Winkel zueinander stehen und das Implantat so ausgelegt ist, dass es nicht in den Wirbelkanal hineinragt, wenn es zwischen dem ersten und dem zweiten Knochenende eingefügt wird,
**dadurch gekennzeichnet, dass** das Körperteil im wesentlichen röhrenförmig ist und aus einem allogenen Knochen-Transplantat-Material hergestellt ist.

2. Implantat nach Anspruch 1, wobei der innere Seitenbereich und die jeweiligen Knocheneingriffsteile an ihrem Schnittpunkt einen Winkel einschließen.

3. Implantat nach Anspruch 2, wobei der Winkel im Bereich von ungefähr 50 bis ungefähr 70 Grad liegt und die innere Seitenlänge im Bereich von ungefähr 6 bis ungefähr 10 Millimeter liegt.

4. Implantat nach einem der Ansprüche 1 bis 3, wobei der Umfang der Außenoberfläche (3) des Implantats eine im Wesentlichen geometrische Form ist.

5. Implantat nach Anspruch 4, wobei die geometrische Form eine Ellipse mit einer Breite (W) und einer Tiefe (D) ist oder die geometrische Form ein Kreis ist.

6. Implantat nach Anspruch 5, wobei die Breite im Bereich von ungefähr 10,0 bis ungefähr 11,5 Millimeter liegt und die Tiefe im Bereich von ungefähr 6,5 bis ungefähr 7,5 Millimeter liegt.

7. Implantat nach einem der Ansprüche 1 bis 6, wobei die Oberflächenfortsätze Sägezahngrate umfassen oder wobei die Oberflächenfortsätze einzelne Pyramidenzähne umfassen.

8. Implantat nach einem der Ansprüche 1 bis 7, wobei das erste und das zweite Ende jeweils ferner einen Kanal umfassen, der dazu ausgelegt ist, die Arme einer Distraktor-Zange aufzunehmen.

9. Implantat nach einem der Ansprüche 1 bis 8, wobei das Körperteil ferner mindestens einen hohlen Nahtbefestigungsteil (9) aufweist, um es einem Chirurgen zu ermöglichen, das Implantat an mindestens dem ersten oder dem zweiten Knochenabschnitt zu befestigen.

10. Implantat nach einem der Ansprüche 1 bis 9, wobei mindestens der erste oder der zweite Knocheneingriffsteil entmineralisierte Knochen umfasst.

11. Implantat nach Anspruch 10, wobei die Innenoberfläche (4) durch einen Knochenmarkkanal des Spenderknochens definiert ist.

12. Implantat nach Anspruch 10 oder 11, wobei die Innenoberfläche so ausgelegt ist, dass das Volumen des im Wesentlichen hohlen Teils größer als der Knochenmarkkanal des Spenderknochens ist.

13. Implantat nach einem der Ansprüche 1 bis 9, wobei mindestens ein Teil des Implantats aus einem biokompatiblen Metall oder aus einem biokompatiblen Polymer hergestellt ist.

14. Implantat nach Anspruch 1, wobei das im Wesentlichen röhrenförmige Körperteil ferner eine Breite (W) und eine Tiefe (D) hat und wobei mindestens der erste oder der zweite Knocheneingriffsteil ein entmineralisiertes allogenes Transplantat-Material umfasst.

15. Implantat nach Anspruch 14, wobei das Körperteil ferner eine Wand, die eine Außenoberfläche hat, sowie eine Innenoberfläche aufweist, die einen im Wesentlichen hohlen Teil definiert, wobei der hohle Teil mit dem ersten und dem zweiten Ende in Verbindung steht.

16. Implantat nach Anspruch 15, wobei der hohle Teil (7) durch den Knochenmarkkanal des Spenderknochens definiert ist.

17. Implantat nach Anspruch 15 oder 16, wobei die Innenoberfläche (4) so ausgelegt ist, dass das Volumen des im Wesentlichen hohlen Teils (7) größer als der Knochenmarkkanal des Spenderknochens ist.

18. Implantat nach einem der vorhergehenden Ansprüche 14 bis 17, wobei mindestens der erste oder der zweite Knocheneingriffsteil ferner Oberflächenfortsätze aufweist, die dazu ausgelegt sind, das Implantat innerhalb des ersten und des zweiten Knochenabschnitts zu halten.

19. Implantat nach Anspruch 14, wobei die Oberflächenfortsätze Sägezahngrate umfassen oder wobei die Oberflächenfortsätze einzelne Pyramidenzähne umfassen.

20. Implantat nach einem der Ansprüche 14 bis 19, wobei der innere Seitenbereich (3B) und jeweils der erste und der zweite Knocheneingriffsteil an ihrem Schnittpunkt einen Winkel einschließen.

21. Implantat nach Anspruch 20, wobei der Winkel im Bereich von ungefähr 50 bis ungefähr 70 Grad liegt, die innere Seitenlänge im Bereich von ungefähr 6 bis ungefähr 10 Millimeter liegt, die Breite (W) im Bereich von ungefähr 10 bis ungefähr 11,5 Millimeter liegt und die Tiefe (D) im Bereich von ungefähr 6,5 bis ungefähr 7,7 Millimeter liegt.

22. Implantat nach Anspruch 1, wobei der mindestens eine Knocheneingriffsteil ferner einen äußeren Schalenteil umfasst, der einen Mittelbereich im Wesentlichen umgibt, wobei der äußere Schalenteil Rindenknochen und der Mittelteil Knochenschwammsubstanz aufweist.

23. Implantat nach Anspruch 22, wobei die Oberflächenfortsätze Sägezahngrate umfassen oder wobei die Oberflächenfortsätze einzelne Pyramidenzähne umfassen.

24. Implantat nach Anspruch 22 oder 23, wobei mindestens die erste oder die zweite Knocheneingriffsfläche entmineralisierte Knochen umfassen.

25. Implantat nach Anspruch 22 oder 23, wobei die Innenseite und der mindestens eine Knocheneingriffsteil an ihrem Schnittpunkt einen Winkel einschließen.

26. Implantat nach Anspruch 25, wobei der Winkel im Bereich von ungefähr 50 bis ungefähr 70 Grad liegt und die innere Seitenlänge im Bereich von ungefähr 6 bis ungefähr 10 Millimeter liegt.

27. Implantat nach einem der vorhergehenden Ansprüche 22 bis 26, wobei das Körperteil ferner einen Nahtbefestigungsteil (9) aufweist, um es einem Chirurgen zu ermöglichen, das Implantat an mindestens dem ersten oder dem zweiten Knochenabschnitt zu befestigen.

## Revendications

1. Implant (1) destiné à être utilisé pour maintenir une distance souhaitée entre des première et seconde extrémités osseuses diviser en deux d'une colonne vertébrale, ledit implant comprenant :
une partie de corps ayant une longueur (L) et configurée pour être insérée entre les première et seconde extrémités osseuses de l'apophyse épineuse d'une seule vertèbre,
la partie de corps ayant une surface externe (3), et une surface interne (4) définissant une partie sensiblement creuse (7), la partie de corps ayant en outre une région latérale interne (3B) ayant une longueur latérale interne, et les première (6A) et seconde (6B) extrémités qui communiquent avec ladite partie creuse, les première (6A) et seconde (6B) extrémités comprenant des parties de mise en prise d'os, au moins l'une des parties de mise en prise d'os comprend des saillies de surface pour réduire le glissement entre les parties de mise en prise d'os et ledit segment osseux respectif,
dans lequel lesdites parties de mise en prise d'os sont coudées l'une par rapport à l'autre, et ledit implant est configuré afin de ne pas faire saillie dans le canal rachidien lorsqu'il est inséré entre les première et seconde extrémités osseuses, **caractérisé en ce que** ladite partie de corps est sensiblement tubulaire et formée avec une matière d'allogreffe osseuse.

2. Implant selon la revendication 1, dans lequel l'intersection entre la région latérale interne et chacune des parties de mise en prise d'os comprend un angle.

3. Implant selon la revendication 2, dans lequel l'angle est de l'ordre d'environ 50 à environ 70 degrés et la longueur latérale interne est de l'ordre d'environ 6 à environ 10 millimètres.

4. Implant selon l'une quelconque des revendications 1 à 3 précédentes, dans lequel le périmètre de la surface externe (3) de l'implant a une forme sensiblement géométrique.

5. Implant selon la revendication 4, dans lequel la forme géométrique est une ellipse ayant une largeur (W) et une profondeur (D) ou la forme géométrique est un cercle.

6. Implant selon la revendication 5, dans lequel la largeur est de l'ordre d'environ 10,0 à environ 11,5 millimètres et la profondeur est de l'ordre d'environ 6,5 à environ 7,5 millimètres.

7. Implant selon l'une quelconque des revendications 1 à 6 précédentes, dans lequel les surfaces de saillie comprennent des crêtes en dents de scie ou dans lequel les saillies de surface comprennent des dents pyramidales individuelles.

8. Implant selon l'une quelconque des revendications 1 à 7 précédentes, dans lequel chacune parmi la première et la seconde extrémité comprend en outre un canal configuré pour accepter les bras d'une paire de pinces d'écarteur.

9. Implant selon l'une quelconque des revendications 1 à 8 précédentes, dans lequel la partie de corps comprend en outre une partie de fixation de suture creuse (9) pour permettre à un chirurgien de fixer l'implant sur au moins l'un parmi lesdits premier et second segments osseux.

10. Implant selon l'une quelconque des revendications 1 à 9 précédentes, dans lequel au moins l'une parmi les première et seconde parties de mise en prise d'os est composée d'os déminéralisé.

11. Implant selon la revendication 10, dans lequel la surface interne (4) est définie par le canal intermédullaire de l'os donneur.

12. Implant selon la revendication 10 ou 11, dans lequel ladite surface interne est configurée de sorte que le volume de ladite partie sensiblement creuse est supérieur au canal intermédullaire de l'os donneur.

13. Implant selon l'une quelconque des revendications 1 à 9, dans lequel au moins une partie de l'implant est fabriquée avec un métal biocompatible, ou avec un polymère biocompatible.

14. Implant selon la revendication 1, dans lequel la partie de corps sensiblement tubulaire comprend en outre une largeur (W) et une profondeur (D) et dans lequel au moins l'une parmi les première et seconde parties de mise en prise d'os est composée d'un matériau d'allogreffe déminéralisé.

15. Implant selon la revendication 14, dans lequel la partie de corps comprend en outre une paroi ayant une surface externe et une surface interne définissant une partie sensiblement creuse, dans lequel la partie creuse est en communication avec les première et seconde extrémités.

16. Implant selon la revendication 15, dans lequel ladite partie creuse (7) est définie par le canal inter médullaire de l'os donneur.

17. Implant selon les revendications 15 ou 16, dans lequel ladite surface interne (4) est configurée de sorte que le volume de ladite partie sensiblement creuse (7) est supérieur au canal intermédullaire de l'os donneur.

18. Implant selon l'une quelconque des revendication 14 à 17 précédentes, dans lequel ladite au moins une parmi lesdites première et seconde parties de mise en prise d'os comprend en outre des saillies de surface configurées pour retenir ledit implant à l'intérieur des premier et second segments osseux.

19. Implant selon la revendication 14, dans lequel les saillies de surface comprennent des crêtes en dents de scie, ou dans lequel les saillies de surface comprennent des dents pyramidales individuelles.

20. Implant selon l'une quelconque des revendications 14 à 19, dans lequel l'intersection entre la région latérale interne (3B) et chacune des première et seconde parties de mise en prise d'os comprend un angle.

21. Implant selon la revendication 20, dans lequel l'angle est de l'ordre d'environ 50 à environ 70 degrés, la longueur latérale interne est de l'ordre d'environ 6 à environ 10 millimètres, la largeur (W) est de l'ordre d'environ 10 millimètres à environ 11,5 millimètres, et la profondeur (D) est de l'ordre d'environ 6,5 millimètres à environ 7,7 millimètres.

22. Implant selon la revendication 1, dans lequel ladite au moins une partie de mise en prise d'os comprend en outre une partie de coque externe entourant sensiblement une région centrale, dans lequel la partie de coque externe est l'os cortical et la région centrale est l'os spongieux.

23. Implant selon la revendication 22, dans lequel les saillies de surface comprennent des crêtes en dents de scie, ou dans lequel les saillies de surface comprennent des dents pyramidales individuelles.

24. Implant selon la revendication 22 ou 23, dans lequel au moins l'une parmi les première et seconde faces de mise en prise d'os est composée d'os déminéralisé.

25. Implant selon la revendication 22 ou 23, dans lequel l'intersection entre le côté interne et ladite au moins une partie de mise en prise d'os comprend un angle.

26. Implant selon la revendication 25, dans lequel l'angle est de l'ordre d'environ 50 à environ 70 degrés et la longueur latérale interne est de l'ordre d'environ 6 à environ 10 millimètres.

27. Implant selon l'une quelconque des revendications 22 à 26 précédentes, dans lequel la partie de corps comprend en outre une partie de fixation de suture creuse (9) pour permettre à un chirurgien de fixer l'implant sur au moins l'un parmi lesdits premier et second segments osseux.
